# EUROPEAN PATENT APPLICATION

(11) **EP 4 488 279 A1**
(43) Date of publication of application: **08.01.2025**
(21) Application number: 23382682.5
(22) Date of filing: 03.07.2023
(51) Int. Cl.: C07D 487/04, A61P 35/00

(54) **SUBSTITUTED HEXAHYDRO-2H-PYRAZINO[1,2-A]PYRAZIN-1(6H)-ONE DERIVATIVES AS ACTIVATORS OF HUMAN CASEINOLYTIC PROTEASE P (HSCLPP)**

(71) Applicant: Centro Atlantico del Medicamento S.A (Ceamed, S.A), 38204 Santa Cruz de Tenerife (ES)
(72) Inventor: McNaughton-Smith, Grant, 38740 Los Silos, Tenerife (ES); Ayra Plasencia, Jessel, 38009 La Florita (Santa Cruz de Tenerife) (ES); Velázquez García, Silvia Ascensión, 38007 Santa Cruz de Tenerife (ES); Estévez Silva, Héctor Martín, 38005 Santa Cruz de Tenerife (ES)
(74) Representative: Manuel Illescas y Asociados, S.L.

(57) **Abstract**

Substituted hexahydro-2H-pyrazino[1,2-a]pyrazin-1(6H)-one derivatives of formula (I) that activate human caseinolytic protease P (HsCIpP), and pharmaceutical compositions comprising such compounds, are disclosed. Additionally, methods of treating or alleviating conditions such as cancer, through the modulation of HsCIpP, are also disclosed.

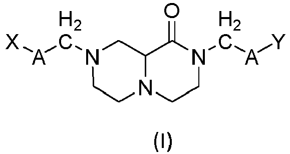

## Description

### FIELD OF THE INVENTION

The present invention discloses compounds of formula (I), and pharmaceutical compositions containing formula (I), and their application as pharmaceuticals for the treatment of diseases. In particular, the invention discloses compounds of formula (I), their preparation and their use in treatments of a disease or condition responsive to activation of human caseinolytic protease P (HsCIpP).

### BACKGROUND OF THE INVENTION

Proteolysis is an essential mechanism for eukaryotic cells. It allows them to maintain protein homeostasis, or modify protein levels, in order to adapt to their changing environment. This proteolysis is carried out by a group of specialized enzymes called proteases, among which is the caseinolytic protease P (CIpP). This protease CIpP, along with chaperone proteins, form a serine protease complex. Seven monomer CIpP units initially co-assemble to form a donut shaped entity. Two of these entities stack one upon the other to form a barrel shape with a central pore. The chaperones bind to one, or both, ends of this tetradecameric structure, to complete the CIpP-CIpPATPase protease system. The role of the chaperones is to bind damaged or misfolded proteins and feed them into the tetradecameric barrel shaped ClpP complex, which degrades them. In human cells, human CIpP (HsCIpP) forms a complex with CIpXP chaperones, and the complex is located in the mitochondrial matrix. This complex participates in the maintenance of the mitochondrial proteome through the degradation of misfolded or dysfunctional proteins (1). This guarantees the proper functioning of the mitochondria, which regulates the bioenergetic activity of a cell (1).

Cancer cells need to generate higher quantities of proteins to support their prolonged growth/proliferation phases. To remove damaged or badly constructed proteins, that would be detrimental to these cells, they require elevated quantities of proteases to remove them. HsCIpP expression levels have been reported to be considerably higher in several cancer cells compared to normal cells (2). Dysregulation of these well controlled proteolytic processes, by either inhibition or activation of their function, is detrimental to the well-being of these cells, and therefore maybe useful mechanism of action against cancer (3).

A few naturally occurring, and synthetic, compounds have been identified as activators of HsCIpP (4-7). Activators of HsCIpP induce significant conformational changes in the tetrameric structure, which lead to the loss of binding of the chaperones, and the stabilization of the axial pore in an open and active position. These combined events allow the HsCIpP system to act independently of the chaperones, and to degrade a greater range of proteins. In human cells with elevated HsCIpP levels, these agonists promote ClpP-mediated degradation of respiratory chain complexes, activating the JNK/c-Jun pathway and inducing an endoplasmic reticulum (ER) stress response. This response consequently leads to the activation of apoptopic pathways and causes growth arrest. Known activators of HsCIpP, such as the acyldepsipeptides (ADEPs), TR-57, ZG-111, ONC-201 and ONC-206 have been reported to reduce cell viability in several human derived cancer cells including pancreatic, AML, glioma, glioblastoma, colorectal, ovarian and breast cancers Furthermore, several of these compounds have been reported to reduce tumour growth in *in vivo* models of cancers. Currently, the small molecules ONC-201 and ONC-206, are being studied in human clinical trials against several type of cancers.

Herein we describe a family of substituted hexahydro-2H-pyrazino[1,2-a]pyrazin-1(6H)-one derivative compounds and their preparation. These compounds activate HsClpP and reduce the cell viability and cell growth of several types of cancer cells.

### BRIEF DESCRIPTION OF THE INVENTION

The present invention relates to compounds which act as activators of human caseinolytic protease P (HsCIpP), wherein said compounds are characterized for having a formula (I), or a pharmaceutically acceptable salt thereof: 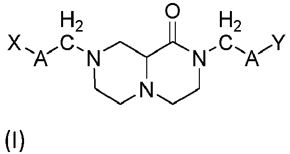 wherein
A is independently selected from the group consisting of: C₁-C₆ alkyl or is absent;
X and Y are independently selected from the group consisting of C₁-C₆ haloalkyl, aromatic carbocyclic 6-membered ring, or an aromatic heterocyclic 5- or 6-membered ring comprising one or more heteroatoms selected from the group consisting of O, N and S, wherein said aromatic carbocyclic ring, or said aromatic heterocyclic ring, is unsubstituted or substituted at one or more atoms of C, with a R⁷ substituent;
R⁷ substituent is independently selected from the group consisting of: H, halogen, C₁-C₆ haloalkyl, -O-C₁-C₆ alkyl, -O-C₁-C₃ haloalkyl, cyano, C₁-C₆ alkyl and -C(O)OR⁸ ;
R⁸ is independently selected from the group consisting of: C₁-C₆ alkyl, C₃-C₆ cycloalkyl or C₁-C₆ aminoalkyl;
When A is absent X and/or Y are directly bound to the neighbouring CH₂ group.

The present invention also refers to a compound of formula (I), according to the present invention, or to a pharmaceutically acceptable salt thereof, for use as a medicament and, in particular, for use in the prevention and/or treatment of a disease or condition responsive activation of CIpP, in particular, for use in the prevention and/or treatment of cancer.

Finally, the present invention also refers to a pharmaceutical composition comprising at least one compound of formula (I), according to the present invention, or a pharmaceutically acceptable salt thereof, and at least one pharmaceutically acceptable excipient, for use as a medicament and, in particular, for use in the prevention and/or treatment of a disease or condition responsive to activation of CIpP, particularly for use as medicaments in the prevention and/or treatment of cancer.

### BRIEF DESCRIPTION OF THE FIGURES

Figure 1: Representative comparison of the growth of yeast without HsCIpP (a), and in yeast expressing HsCIpP (b), in the presence of various concentrations of the known CIpP agonist ONC-206, and compounds of formula (I), (4), (6), (8), (9) and (12). Optical density was used as the measure of yeast growth. GI50 values represent the concentration of compound needed to reduce the growth by 50%. Selective activators of HsCIpP will only reduce the growth of yeasts expressing HsCIpP.
Figure 2: Cell viability study in human derived MDA-MB-231 triple negative breast cancer cells. Compounds ONC-201, ONC-206, (1), (3), (5) and (12) were incubated with MDA-MB-231 cells are varying concentrations for 72 h. Cell viability was measure using the MTT assay. IC50 values represent the concentration of compound needed to reduce the viability by 50%.
Figure 3: ONC-206, and compounds of the invention (12) and (13), reduce the growth of MDA-MB-231 cells, as measured by the SRB assay, in a concentration dependent manner.
**Figure 4:** (a) Time-dependent growth, of established 4T1 tumours, treated with either vehicle, or compound (4), and (b) body weights during the experimental period.

### DETAILED DESCRIPTION

The present invention refers to compounds of formula (I), or a pharmaceutically acceptable salt thereof, as disclosed herein, which activate the proteolytic functions of the human caseinolytic protease protein (HsCIpP). Compounds of formula (I) according to the present invention are compounds comprising of a hexahydro-2H-pyrazino[1,2-a]pyrazin-1(6H)-one, core moiety, bearing two appendages at the non-bridgehead nitrogens, and are characterized by having a formula (I): 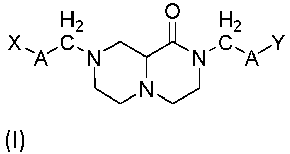 wherein
A is independently selected from the group consisting of: C₁-C₆ alkyl or is absent;
X and Y are independently selected from the group consisting of C₁-C₆ haloalkyl, aromatic carbocyclic 6-membered ring, or an aromatic heterocyclic 5- or 6-membered ring comprising one or more heteroatoms selected from the group consisting of O, N and S, wherein said aromatic carbocyclic ring, or said aromatic heterocyclic ring, is unsubstituted or substituted at one or more atoms of C, with a R⁷ substituent;
R⁷ substituent is independently selected from the group consisting of: H, halogen, C₁-C₆ haloalkyl, -O-C₁-C₆ alkyl, -O-C₁-C₃ haloalkyl, cyano, C₁-C₆ alkyl and -C(O)OR⁸;
R⁸ is independently selected from the group consisting of: C₁-C₆ alkyl, C₃-C₆ cycloalkyl or C₁-C₆ aminoalkyl.

Present disclosure refers also to the pharmaceutically acceptable salts, solvates and polymorphs of the compounds of formula (I) described according to the present invention.

The term "pharmaceutical acceptable" is employed herein to refer to those compounds, materials, compositions, and/or dosage forms which, when contacted with the tissue of human beings or animals, possess a benefit/risk ratio greater than 1. As used herein "pharmaceutically acceptable salts" refers to derivatives of the disclosed compounds wherein the parent compound is modified by converting an existing acid or base moiety to its salt form. Examples of pharmaceutically acceptable salts include, but are not limited to:
- mineral or organic acid salts of basic residues such as amines. Examples of mineral acid salts of basic residues include hydrochloride, hydrobromide, hydroiodide, phosphate and sulfate salts. Examples of organic acid salts of basic residues include tartrate (e.g. (+)-L-tartrate), maleate, citrate, acetate, lactate, mesylate, methylsulfate and fumarate salts;
- alkali or organic salts of acidic residues such as carboxylic acids. Examples of alkali salts of acidic residues include sodium, potassium, calcium, magnesium, and ammonium salts. Examples of organic salts of acidic residues include L-argininate, N-methylglucaminate, cholinate, lysinate, benethaminate, diethylaminate, 2-diethylaminoethanol base, 4-(2-hydroxyethyl)morpholine base, 1-(2-hydroxyethyl)pyrrolidine base and trimethaminate base salts.

The salts may be formed by conventional means, such as by reacting the free base form of the compound with one or more equivalents of the appropriate acid in a solvent or medium in which the salt is insoluble, or in a solvent such as water which can be removed in vacuo or by freeze drying or by exchanging the anions of an existing salt for another anion using a suitable ion exchange resin.

The term "solvate" in accordance with this invention should be understood as meaning any form of the active compound in accordance with the invention in which said compound is bonded by a non-covalent bond to another molecule (normally a polar solvent), including especially hydrates and alcoholates.

The term "polymorph" refers to crystalline forms having the same chemical composition but different spatial arrangements of the molecules, atoms, and/or ions forming the crystal. Certain compounds of the present invention can exist in unsolvated forms as well as solvated forms, including hydrate forms. Certain compounds of the present invention may exist in multiple crystalline (polymorph) or amorphous forms.

The abbreviations used herein have their conventional meaning within the chemical and biological arts. For example, DMSO, dimethylsulfoxide; MeOH, methanol; RPMI medium, Roswell Park Memorial Institute medium; THF, tetrahydrofuran; DCM, dichloromethane; EtOAc, ethyl acetate, EtOH, ethanol; MTT, 3-(4,5-methyltiazol-2yl)-2,5diphenyl-tetrazolium bromide]; FBS, Fetal bovine serum; MgSO₄, Magnesium sulphate anhydrous; HCl, hydrochloric acid, H₂SO₄, sulfuric acid, AcOH, acetic acid; ATCC, American Type Culture Collection; DMEM, Dulbecco's Modified Eagle's Medium.

For the purposes of present invention, the term "comprises" includes any of the terms "consist of", or "consists essentially of". Accordingly, "comprises" may refer to a group of features A, B and C, which additionally may include other features, such as E and D, with the condition that said features do not render the claim unworkable, but said term "comprises" also includes the situation in which the group of features "consists of" or "consists essentially" of A, B and C.

The term "C₁-C₆ alkyl" refers to straight chain or branched chain hydrocarbon groups having from 1 to 6 carbon atoms. Examples include methyl, ethyl, propyl, isopropyl, butyl, isobutyl, sec-butyl, tert-butyl, pentyl, neopentyl and hexyl, preferably ethyl, propyl, isopropyl, n-butyl, isobutyl, sec-butyl, tert-butyl, pentyl, neopentyl and hexyl, more preferably ethyl, propyl, isopropyl, n-butyl, tert-butyl or pentyl.

The term "cycloalkyl group" refers to a non-aromatic carbocycle or cyclic hydrocarbon group. Examples include cyclopropyl, cyclobutyl, cyclopentyl, cyclopentenyl, cyclohexyl, cyclohexenyl, cycloheptyl, norbornyl and adamantly.

The term "C₁-C₆ aminoalkyl" refers to straight chain or branched chain hydrocarbon groups having from 1 to 6 carbon atoms and bearing an amino (-NH₂) or substituted amino group. Examples include CH₂NH₂, CH₂NHCH₃, CH₂N(CH₃)₃, CH₂NHCH₂CH₃, CH₂CH₂NH₂, CH₂CH₂NHCH₃, CH₂CH₂N(CH₃)₂, CH(NH₂)CH₃, CH(NHCH₃)CH₃, CH₂C[N(CH₃)₂]CH₃, CH₂CH(NH₂)CH₃, CH₂CH(NHCH₃)CH₃, CH₂CH₂CH₂NH₂.

The term "C₃-C₆ aminocycloalkyl" refers to cyclic hydrocarbon chain that bears an amino (-NH₂) or substituted amino group. Examples include cyclopropanamine, cyclobutanamine, cyclopentanamine, cyclohexanamine, N-methylcyclopropanamine, N-methylcyclobutanamine, N-methylcyclopentanamine, N-methylcyclohexanamine, N,N-dimethylcyclopropanamine, N,N-dimethylcyclobutanamine, N,N-dimethylcyclopentanamine, N,N-dimethylcyclohexanamine.

The terms "heterocyclic group" or "heterocycloalkyl group" refer both indistinctively to non-aromatic carbocycles having one or more ring forming heteroatoms such as N, O and S atoms. Heterocyclic groups may include monocyclic and polycyclic (e.g., having 2, 3 or 4 fused rings) systems as well as spirocycles. Example groups include morpholino, thiomorpholino, piperazinyl, tetrahydrofuranyl, tetrahydrothienyl, 2,3-dihydrobenzofuryl, piperidinyl and pyrrolidinyl. Ring forming carbon atoms and heteroatoms of a heterocyclic group can be optionally substituted by oxo or sulfido. Also included in the definition of heterocyclic group are moieties that have one or more aromatic rings fused (i.e., having a bond in common with) to the non-aromatic heterocyclic ring. Examples of heterocyclic groups include without limitation, phthalimidyl, naphthalimidyl, and benzo derivatives of heterocycles. The heterocyclic group can be attached through a ring-forming carbon atom or a ring-forming heteroatom. The number of carbon atoms present in the heterocyclic group can range from 1 to 20 and may include double or triple bonds.

The terms "aryl group" or "aromatic carbocyclic group" refer both indistinctively to single, polynuclear, conjugated or fused residues of cyclic aromatic hydrocarbons, including both substituted or unsubstituted versions thereof. Examples of "aryl groups" or "aromatic carbocycle groups" include: phenyl, biphenyl, naphthyl, tetrahydronaphthyl, anthracenyl, benzanthracenyl, phenylanthrenyl, nitrophenyl, chlorophenyl, bromophenyl, fluorophenyl, iodophenyl, benzo[d][1,3]dioxol-5-yl, alkylphenyls, alkoxyphenyl, hydroxyphenyl, aminophenyl, cyanophenyl and mercaptophenyl, preferably 3-nitrophenyl, 4-nitrophenyl, 3-fluorophenyl, 4-fluorophenyl, 3-iodophenyl, 4-iodophenyl, ethylphenyl, isopropylphenyl, 3-benzamide, 4-benzamide, aminophenyl, 3-cyanophenyl, 4-cyanophenyl and mercaptophenyl, more preferably 4-nitrophenyl, 4-cyanophenyl, 4-benzamide and 3-cyanophenyl.

The term "heteroaryl group" or "aromatic heterocyclic group" refers to an aromatic carbocycle having at least one heteroatom. As used herein, the term "heteroatom" is meant to include oxygen (O), nitrogen (N), sulphur (S), silicon (Si) and phosphorus (P); preferably, said heteroatom is S, N or O. Heteroaryl groups include monocyclic and polycyclic systems. Examples of heteroaryl groups include without limitation, pyridyl, pyrimidinyl, pyrazinyl, pyridazinyl, triazinyl, furyl, quinolyl, isoquinolyl, thienyl, imidazolyl, thiazolyl, indolyl, pyrolyl, benzofuryl, benzothienyl, isoxazolyl, pyrazolyl, triazolyl, tetrazolyl, indazolyl, carbazolyl, benzimidazolyl and indolinyl, preferably pyridyl, pyrimidinyl, pyrazinyl, pyridazinyl, triazinyl, furyl, quinolyl, isoquinolyl, imidazolyl, thiazolyl, indolyl, pyrolyl, benzofuryl, benzothienyl, isoxazolyl, pyrazolyl, triazolyl, tetrazolyl, indazolyl, carbazolyl, benzimidazolyl and indolinyl. In some embodiments, any ring forming N can be substituted by an oxo, to form an N-oxide.

The term "halogen" refers to fluorine, chlorine, bromine and iodine. The terms "amino", "azido", "nitro", "cyano" and "hydroxyl" respectively refer to -NH₂, -N₃, - NO₂, -CN and -OH groups attached to the remainder of the molecule.

For the purpose of present disclosure the dash "-" featured in a group listed is linked to the atom by which said group is attached to the reminder of the molecule. For example, for the purposes of the present disclosure, the group -OC(O)CH₃ is an ester group which is attached to the reminder of the molecule by the oxygen atom, whereas the group -C(O)OCH₃ is an ester group which is attached to the reminder of the molecule by the carbon atom of the carbonyl moiety. When the "-" is not indicated in a group, it may be linked to the reminder of the molecule by any of the atoms which are capable of forming a bond.

The term "substituted or unsubstituted" refers to a group that may or may not be further substituted with one or more groups selected from (C₁-C₆)alkyl, Si[(C₁-C₆)alkyl]₃, (C₃-C₈)cycloalkyl, (C₂-C₆)alkenyl, (C₂-C₆)alkynyl, aryl, heteraryl, heterocyclyl, halogen, (C₁-C₆)alkoxyl, amino, amido, azido, nitro, cyano, carboxy, sulphonamide, urea, sulphone, acyl, mercapto, alkylamino, alkylthio and hydroxyl. Preferably, said term "substituted or unsubstituted" refers to a group that may or may not be further substituted with one or more groups selected from (C₁-C₆)alkyl, Si(C₁-C₆)alkyl)₃, (C₃-C₈)cycloalkyl, (C₂-C₆)alkenyl, (C₂-C₆)alkynyl, aryl, heteraryl, heterocyclyl, halogen, (C₁-C₆)alkoxyl, amino, azido, nitro, cyano, carboxy, sulphonamide, urea, sulphone, acyl, mercapto and hydroxyl. In one embodiment said term "substituted or unsubstituted" refers to a group that may or may not be further substituted with one or more groups selected from (C₁-C₆)alkyl, Si(C₁-C₆)alkyl)₃, (C₁-C₆)cycloalkyl, (C₂-C₆)alkenyl, (C₂-C₆)alkynyl, aryl, heteraryl, heterocyclyl, halogen, (C₁-C₆)alkoxyl, amino, azido, nitro, cyano, carboxy, sulphonamide, urea, sulphone, acyl, mercapto, and the like. In an alternative embodiment "substituted or unsubstituted" refers to a group that may or may not be further substituted with one or more groups selected from (C₂-C₆)alkyl, Si(C₁-C₆)alkyl)₃, (C₃-C₈)cycloalkyl, (C₂-C₆)alkenyl, (C₂-C₆)alkynyl, aryl, heteraryl, heterocyclyl, (C₂-C₆)alkoxyl, amino, azido, cyano, carboxy, sulphonamide, urea, sulphone, acyl and mercapto groups. In another embodiment "substituted or unsubstituted" refers to a group that may or may not be further substituted with one or more groups selected from (C₂-C₆)alkyl, (C₂-C₆)alkenyl, (C₂-C₆)alkoxyl, amino, cyano, carboxy, acyl, mercapto and hydroxyl groups, further preferably (C₂-C₆)alkyl, (C₂-C₆)alkenyl, (C₂-C₆)alkoxyl, amino, cyano, carboxy, acyl and mercapto groups.

A particular preferred embodiment of the present invention refers to a compound of formula (I) according to the present invention, or a pharmaceutically acceptable salt or solvate thereof: 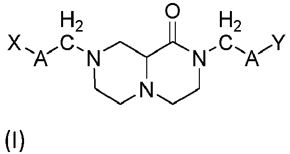 wherein
A is independently selected from the group consisting of: C₁-C₆ alkyl or is absent;
X and Y are independently selected from the group consisting of C₁-C₆ haloalkyl, aromatic carbocyclic 6-membered ring, or an aromatic heterocyclic 5- or 6-membered ring comprising one or more heteroatoms selected from the group consisting of O, N and S, wherein said aromatic carbocyclic ring, or said aromatic heterocyclic ring, is unsubstituted or substituted at one or more atoms of C, with a R⁷ substituent;
R⁷ substituent is independently selected from the group consisting of: H, halogen, C₁-C₆ haloalkyl, -O-C₁-C₆ alkyl, -O-C₁-C₂ haloalkyl, cyano and C₁-C₆ alkyl.

An even more preferred embodiment of present invention refers to a compound of formula (I) according to the present invention, or a pharmaceutically acceptable salt thereof: 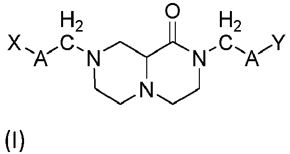 wherein
A is independently selected from the group consisting of: C₁-C₃ alkyl or is absent;
X and Y are independently selected from the group consisting of C₁-C₃ haloalkyl, aromatic carbocyclic 6-membered ring, or an aromatic heterocyclic 5-membered ring comprising one or more heteroatoms selected from the group consisting of O, N and S, wherein said aromatic carbocyclic ring, or said aromatic heterocyclic ring, is unsubstituted or substituted at one or more atoms of C, with a R⁷ substituent;
R⁷ substituent is independently selected from the group consisting of: H, F, Cl, Br, C₁-C₃ haloalkyl, -O-C₁-C₆ alkyl, -O-CF3, cyano and C₁-C₆ alkyl.

Also within the scope of the present invention are compounds of the invention that are attached to an oligomeric or polymeric framework (e.g., polylysine, dextran, hydroxyethyl starch and a like).

The compounds of formula (I) may also be functionalized to afford compounds having a water-solubility that is enhanced relative to analogous compounds that are not similarly functionalized. Methods of enhancing the water-solubility of organic compounds are known in the art. Such methods include, but are not limited to, functionalizing an organic nucleus with a permanently charged moiety, e.g. quaternary ammonium, or a group that is charged at a physiologically relevant pH, e.g. carboxylic acid, amine. Other methods include, appending to the organic nucleus hydroxyl- or amine-containing groups, e.g. alcohols, polyols, polyethers and the like. Representative examples include, but are not limited to, polylysine, polyethyleneimine, poly(ethyleneglycol) and poly(propyleneglycol). Suitable functionalization chemistries and stragies for these compounds are known in the art. See, for example, Dunn, R.L., et al., Eds. POLYMERIC DRUGS AND DRUG DELIVERY SYSTEMS, ACS Symposium Series Vol. 469, American Chemical Society, Washington, D.C. 1991 (ref. 28).

Also disclosed herein are metabolites of the compounds of formula (I), according to present invention, which are compounds formed *in vivo* upon administration of the drug. Some examples of metabolites in accordance with the invention include:
(i) where the compound of formula I contains a methyl group, an hydroxymethyl derivative thereof (-CH₃→ -CH₂OH);
(ii) where the compound of formula I contains an alkoxy group, a hydroxyl derivative thereof (-OR →-OH);
(iii) where the compound of formula I contains a tertiary amino group,a secondary amino derivative thereof (-NR¹R²→ -NHR¹ or -NHR²);
(iv) where the compound of formula I contains a secondary amino group, a primary amino derivative thereof (-NHR¹→ -NH₂); and
(v) where the compound of formula I contains a phenyl group, a phenol derivative thereof (-Ph→ -PhOH).

The compounds of formula (I) containing one or more asymmetric carbon atoms can exist as two or more stereoisomers. Where a compound of formula (I) contains an alkene or alkenylene group, geometric cis/trans (E/Z) isomers are possible. Where structural isomers are interconvertible via a low energy barrier, tautomeric isomerism ("tautomerism") can occur. This can take the form of proton tautomerism in compounds of formula (I) containing, for example, an imino, keto, or oxime group, or so-called valence tautomerismin compounds which contain an aromatic moiety. It follows that a single compound may exhibit more than one type of isomerism. Thus, the compounds of the present invention also include all stereoisomers, geometric isomers, and tautomeric forms of the compounds of formula (I), as hereinbefore defined, including compounds that exhibit more than one type of isomerism, and mixtures of one or more thereof. Also included are acid or base salts wherein the counter ion is optically active, for example, d-lactate or l-lysine, or racemic, for example, dl-tartrate or dl-arginine. Cis/trans isomers may be separated by conventional techniques well known to those skilled in the art, for example, chromatography or fractional crystallization.

Where a compound possesses a stable chiral center the compound can be used as a purified enantiomer or diastereomer, or as a mixture of any ratio of stereoisomers. It is however preferred that the mixture comprises at least 70%, 80%, 90%, 95%, 97.5% or 99% of the preferred isomer. Conventional techniques for the preparation/isolation of individual enantiomers include chiral synthesis from a suitable optically pure precursor or enantiomeric enrichment by resolution of the racemate (or the racemate of a salt or derivative) using for example, chiral high pressure liquid chromatography (HPLC), namely using HPLC with an asymmetric resin and a mobile phase. Alternatively, the racemate (or a racemate precursor) may be reacted with a suitable optically active compound, for example, an alcohol, or in the case where the compounds of formula I contains an acidic or basic moiety, a base or acid such as 1-phenylethylamine or tartaric acid. The resulting diastereomeric mixture may be separated by chromatography and/or fractional crystallization, and one or both, of the diastereoisomers converted to the corresponding pure enantiomer(s) by means well known to those skilled in the art.

Also disclosed are pharmaceutically acceptable isotopically labelled compounds of formula (I), as hereinbefore defined, wherein one or more atoms are replaced by atoms having the same atomic number, but an atomic mass or mass number different from the atomic mass or mass number which predominates in nature. Example of isotopes acceptable for inclusion in the compounds of the invention include isotopes of hydrogen such as ²H and ³H, carbons such as ¹¹C, ¹³C and ¹⁴C, chlorine such as ³⁶Cl, fluorine such as ¹⁸F, iodine such as ¹²³I and ¹²⁵I, nitrogen such as ¹³N and ¹⁵N, oxygen such as ¹⁵O, ¹⁷O and ¹⁸O, phosphorus such as ³²P, and sulphur such as ³⁵S. Certain isotopically-labelled compounds of formula I, for example those incorporating a radioactive isotope, are useful in drug and/or substrate tissue distribution studies. The radioactive isotopes tritium, i.e., ³H, and carbon-14, i.e.¹⁴C are particularly useful for this purpose in view of their ease of incorporation and ready means of detection. Substitution with heavier isotopes such as deuterium i.e. ²H, may afford certain therapeutic advantages resulting from greater metabolic stability, for example, increased in vivo half-life or reduced dosage requirements, and hence may be preferred in some circumstances. Substitution with positron emitting isotopes, such as ¹¹C and ¹⁸F, ¹⁵O and ¹³N, can be useful in Positron Emission Topography (PET) studies for examining substrate receptor occupancy. Isotopically labelled compounds of formula I can generally be prepared by conventional techniques known to those skilled in the art or by processes analogous to those described in the accompanying Examples and preparations section, using an appropriate isotopically labelled reagent in place of the non-labelled reagent previously employed.

The compounds of formula (I) according to the present invention activate human CIpP and in doing so cause an imbalance in the cell's proteome. In the case of human cancer cells that overexpress HsCIpP, this imbalance leads to mitochondria stress and eventually apoptosis.

The compounds of formula (I) according to present invention activate HsCIpP, and as a consequence of this activation, our experimental evidence presented in this application indicates that they possess anticancer properties.

Accordingly, one embodiment of the present invention refers to a pharmaceutical composition comprising at least one compound of formula (I), according to the present invention, or a pharmaceutically acceptable salt thereof, and at least one pharmaceutically acceptable excipient.

Another embodiment of present invention refers to an antibody-drug conjugate comprising a compound of formula (I) and a human or humanized monoclonal or polyclonal antibody.

For the purposes of present invention, the term "antibody-drug conjugate" refers to the coupling of a compound, in particular a compound of formula (I) or a pharmaceutically acceptable salt thereof, according to present invention, with a human or humanized monoclonal or polyclonal antibody such as IgG1, IgG2 or IgG4. In an embodiment the compound of formula (I) and the human or humanized monoclonal or polyclonal antibody are linked by a cleavable or a non-cleavable linker, depending on whether the bond between the antibody and the compound of formula (I) may or may not be degraded or broken down by enzymes.

Yet another embodiment of present invention refers to a pharmaceutical composition comprising at least one antibody-drug conjugate comprising a compound of formula (I), according to the present invention, and at least one pharmaceutically acceptable excipient. Additionally, present invention also refers to a compound of formula (I), or a pharmaceutically acceptable salt thereof, or to an antibody-drug conjugate comprising the same, according to the present invention, for use as a medicament. In particular, one embodiment of present invention refers to a compound of formula (I), or a pharmaceutically acceptable salt thereof, or to an antibody-drug conjugate comprising the same, according to the present invention, for use in the prevention and/or treatment of a disease or condition responsive or ameliorated by activation of caseinolytic protease P (CIpP). Preferably, present invention refers to a compound of formula (I), or a pharmaceutically acceptable salt thereof, or to an antibody-drug conjugate comprising the same, according to the present invention, for use in the prevention and/or treatment of cancer.

Another embodiment disclosed in present invention refers to a pharmaceutical composition comprising at least one compound of formula (I), or a pharmaceutically acceptable salt thereof, or an antibody-drug conjugate comprising the same, according to the present invention, and at least one pharmaceutically acceptable excipient, for use as a medicament and, in particular, for use in the prevention and/or treatment of a disease or condition responsive or ameliorated by activation of caseinolytic protease P (CIpP). Preferably, present invention refers to a pharmaceutical composition comprising at least one compound of formula (I), or a pharmaceutically acceptable salt thereof, or an antibody-drug conjugate comprising the same, according to the present invention, for use in the prevention and/or treatment of cancer.

Also described is the use of a compound of formula (I), or a pharmaceutically acceptable salt thereof, or an antibody-drug conjugate or a pharmaceutical composition comprising the same, according to the present invention, in the manufacturing of a medicament for preventing /and/or treating a disease or condition responsive to activation of caseinolytic protease P (CIpP) and, preferably, for prevention and/or treatment of cancer.

Additionally, described is a method for preventing and/or treating a disease or condition responsive or ameliorated by activation of caseinolytic protease P (CIpP), wherein said method comprises administering an effective amount of at least one compound of formula (I), or a pharmaceutically acceptable salt thereof, or an antibody-drug conjugate or a pharmaceutical composition comprising the same, according to the present invention, to a subject in need thereof and, preferably, for prevention and/or treatment of cancer.

For the purposes of present invention, the term "disease or condition responsive or ameliorated by activation of caseinolytic protease P (CIpP)" refers to a disease or pathological disorder or condition which may be treated with activators of caseinolytic protease P (CIpP), i.e. a disease or pathological disorder or condition the treatment of which benefits or is ameliorated by activation of caseinolytic protease P (CIpP) and, preferably, for prevention and/or treatment of cancer.

For the purposes of present invention said condition responsive or ameliorated by activation of human caseinolytic protease P (HsCIpP) is selected from the group consisting of a hyperproliferative disease, or cancer.

Preferably, said condition responsive or ameliorated by activation of human caseinolytic protease P (HsCIpP) is a cancer. More preferably, said cancer has elevated protein expression levels of caseinolytic protease P.

In a preferred embodiment, said cancer, is selected from the group consisting of: sarcoma, breast cancer, prostate cancer, bone cancer, head and neck cancer, brain tumour, glioma, glioblastoma, colorectal cancer, lung cancer, pancreatic cancer, cervical cancer, ovarian cancer, gastric cancer, renal cell carcinoma, melanoma, endometrial cancer, hepatocellular carcinoma, chronic myelogenous leukaemia, acute myelogenous leukaemia, cutaneous T-cell lymphoma, Hodgkin's disease, anaplastic large-cell lymphoma and Burkitt's lymphoma.

In one embodiment, the pharmaceutical compositions disclosed comprise at least one additional active compound or therapeutic ingredient. Said additional active compound or therapeutic ingredient provides additive or synergistic biological activities. For the purposes of present description, the terms "active compound" or "therapeutic ingredient" should be taken as synonyms and mean a chemical or biological entity which exerts therapeutic effects when administered to human or animal beings. Said active compound or therapeutic ingredient exerts therapeutic effects when administered to human or animal beings, and it may be a cell therapy, a small molecule therapy, immunotherapy, radiotherapy, or an antibiotic, among others.

In a preferred embodiment said additional active compound or therapeutic agent is selected from the group consisting of a CAR-T cell therapy, a CAR-NK cell therapy, an antibody, a HIF pathway inhibitor, and a chemotherapeutic agent. Said additional active compound or additional therapy may be administered at the same time, prior to, subsequently to, or at a different moment than the compound of formula (I) for use according to present invention. Preferably, said chemotherapeutic agent is selected from the group consisting of platinum-based antineoplastic agents, anti-mitotic chemotherapeutic agents, a poly adenosine diphosphate ribose polymerase (PARP) inhibitor, type I topoisomerase inhibitors, type II topoisomerase inhibitors, epothilones, cycloskeletal disruptors, alkylating agents, epothilones, histone deacetylase inhibitors, kinase inhibitors, antifolates, kinase inhibitors, peptide antibiotics, retinoids, vinca alkaloids and thymidylate synthase inhibitors. More preferably, the chemotherapeutic agent is selected from the group consisting of cyclophosphamide, ifosfamide, busulfan, temozolomide, mechlorethamine, chlorambucil, melphalan, dacarbazine, daunorubicin, doxorubicin, daunorubicin, epirubicin, idarubicin, mitoxantrone, valrubicin, paclitaxel, docetaxel, abraxane, taxotere, epothilone, vorinostat, romidepsin, irinotecan, topotecan, camptothecin, exatecan, lurtotecan, etoposide, teniposide, tafluposide, bortezomib, erlotinib, gefitinib, imatinib, vemurafenib, vismodegib, azacitadine, azathioprine, capecitabine, cytarabine, cladribine, fludarabine, doxifluridine, fluorouracil, gemcitabine, hydroxyurea, mercaptopurine, methotrexate, pemetrexed, tioguanine, bleomycin, actinomycin, carboplatin, cisplatin, oxaliplatin, tretinoin, alitretinoin, bexarotene, vinblastine, vincristine, vindesine and vinorelbine.

In another preferred embodiment said additional active ingredient is an antibiotic selected from the group consisting of penicillin G, naficillin, oxacillin, ampicillin, amoxicillin, cefazolin, cephalexin, cephotetan, cefoxitin, ceftriaxone, ceftazidime, cefepime, ertapenem, imipenem, meropenem, ciproflaxacin, levofloxacin, moxifloxacin, amikacin, tobramycin, gentamicin, clindamycin, azithromycin, doxyclycine, vancomycin, rifaximin, linezolid, colistin, rifampicin, and isoniazid.

In yet another preferred embodiment said additional active ingredient is a human or humanized monoclonal or polyclonal antibody.

Preferably said antibody is selected mogamulizumab, blinatumomab, ibritumomab, obinutuzumab, ofatumumab, rituximab, tositumomab, inotuzumab, moxetumomab, brentuximab, gemtuzumab, daratumumab, isatuximab, alemtuzumab, polatuzumab, cetuximab, necitumumab, nimotuzumab, panitumumab, catumaxomab, burosumab, dinutuximab, pertuzumab, trastuzumab, ertumaxomab, mepolizumab, siltuximab, enfortumab, etaracizumab, racotumomab, bevacizumab, denosumab, elotuzumab, olaratumab, ramucirumab, bermekimab, labetuzumab, pemtumomab, tacatuzumab, pembrolizumab, nivolumab, cemiplimab, atezolizumab, avelumab, durvalumab, ipilimumab or any other immunotherapeutic agent.

The compounds of formula (I) and pharmaceutical compositions comprising the same, for use according to present invention may also be administered together, prior to, or subsequently to an additional therapy. Preferably said additional therapy is radiotherapy, immunotherapy, chemotherapy or an antibacterial treatment.

In a preferred embodiment, said compound of formula (I), or said pharmaceutical composition for use according to present invention is administered together with, prior to or subsequently to a radiotherapeutic treatment, a chemotherapeutic treatment, an immunotherapeutic treatment, or an antibacterial treatment.

Additionally, the compounds of formula (I) or pharmaceutically salts and solvates thereof, for use according to present invention may also be administered as an antibody-drug conjugate or administered as part of an antibody-drug conjugate, i.e., administered as an antibody-drug conjugate comprising said compounds of formula (I) or pharmaceutically salts and solvates thereof and a human or humanized monoclonal or polyclonal antibody.

The compounds of the present invention can be prepared and administered in a wide variety of oral, parenteral, and topical dosage forms. Thus, the compounds of the present invention can be administered by injection, that is, intravenously, intramuscularly, intracutaneously, subcutaneously, intraduodenally, or intraperitoneally. Also, the compounds described herein can be administered by inhalation, for example, intranasally. Additionally, the compounds of the present invention can be administered transdermal.

For preparing pharmaceutical compositions from the compounds of the present invention, pharmaceutically acceptable carriers can be either solid or liquid. Solid form preparations include powders, tablets, pills, capsules, cachets, suppositories, and dispersible granules. The term "preparation" is intended to include the formulation of the active compound with encapsulating material as a carrier providing a capsule in which the active component with or without other carriers, is surrounded by a carrier, which is thus in association with it. Similarly, cachets and lozenges are included. Tablets, powders, capsules, pills, cachets, and lozenges can be used as solid dosage forms suitable for oral administration. A solid carrier can be one or more substances, which may also act as diluents, flavouring agents, binders, preservatives, tablet disintegrating agents, or an encapsulating material.

In powders and tablets, preferably containing from 5% to 80% of the active compound, suitable carriers are magnesium carbonate, magnesium stearate, talc, sugar, lactose, pectin, dextrin, starch, gelatine, tragacanth, methylcellulose, sodium carboxymethylcellulose, a low melting wax, cocoa butter, hydroxypropylmethylcellulose, polyethyleneglycol, creamaphor, and the like.

For preparing suppositories, a low melting wax, such as a mixture of fatty acid glycerides or cocoa butter, is first melted and the active component is dispersed homogeneously therein, as by stirring. The molten homogeneous mixture is then poured into convenient sized moulds, allowed to cool, and thereby to solidify.

Liquid preparations include solutions, suspensions, and emulsions, for example, water or water/propylene glycol solutions. For peritoneal injection, liquid preparations can be formulated in solution in aqueous polyethylene glycol solution.

Aqueous solutions suitable for oral use can be prepared by dissolving the active component in water and adding suitable colorants, flavours, stabilizers, and thickening agents as desired. Aqueous suspensions suitable for oral use can be prepared by dispersing the finely divided active component in water with viscous material, such as natural or synthetic gums, resins, methylcellulose, cremaphor, sodium carboxymethylcellulose, and other well-known suspending agents.

Thus, a pharmaceutically acceptable carrier may comprise magnesium carbonate, magnesium stearate, talc, sugar, lactose, pectin, dextrin, starch, gelatine, tragacanth, methylcellulose, sodium carboxymethylcellulose, low melting fatty acid glycerides, cocoa butter, water, propylene glycol, natural or synthetic gums, resins, cremaphor, methylcellulose, and sodium carboxymethylcellulose.

The pharmaceutical preparation is preferably in unit dosage form. In such form the preparation is subdivided into unit doses containing appropriate quantities of the active component. The unit dosage form can be a packaged preparation, the package containing discrete quantities of preparation, such as packeted tablets, capsules and powders in vials or ampoules. Also, the unit dosage form can be a capsule, tablet, cachet, or lozenge itself, or it can be the appropriate number of any of these in packaged form.

The quantity of active component in a unit dose preparation may be varied or adjusted from 0.1 mg to 10000 mg, more typically 1.0 mg to 1000 mg, most typically 10 mg to 500 mg, according to the particular application, and the potency of the active component.

The compounds of the present invention, or their pharmaceutically acceptable salts and solvates or prodrugs, are administered in a therapeutically effective amount which will vary depending upon a variety of factors including the activity of the specific compound employed, the metabolic stability and length of action of the compound, age, body weight, general health, sex, diet, mode and time of administration, rate of excretion, drug combination effects, the severity of the particular disease, and the patients side effect profile towards the compound.

Determination of a therapeutically effective amount of a compound of the invention is well within the capabilities of those skilled in the art.

Patient doses for oral administration of the compounds described herein, which is the preferred mode of administration for prophylaxis treatment of an exemplary disease such as cancer, typically range from about 0.1 mg/day to about 10000 mg/day, more typically from about 1 mg/day to about 1000 mg/day, and most typically 1 mg/day to 500 mg/day. Stated in terms of patient body weight, typical dosages range from about 0.01 to about 150 mg/kg/day, more typically from about 0.1 to about 15 mg/kg/day, and most typically from about 0.5 to about 10 mg/kg/day.

For other modes of administration, dosage amount and interval, can be adjusted individually to provide plasma levels of the administered compound, that are effective for the particular clinical indication being treated.

The method by which the compounds of the present invention are prepared is not critical; compounds of the invention, including salts and solvates and prodrugs thereof, can be prepared using known organic synthesis techniques and can be synthesized according to any of numerous possible synthetic routes.

The reactions for preparing compounds of the invention can be carried out in suitable solvents which can be readily selected by one skilled in the art of organic synthesis. A given reaction can be carried out in one solvent or a mixture of more than one solvent and at temperatures that can range from the solvent's freezing temperature to the solvent's boiling temperature. In cases where sealed tube assisted heating is used then the solvent temperature may exceed the boiling temperature of the solvent.

Preparation of compounds of the invention can involve protection and deprotection of various chemical groups. The need for protection and deprotection, and the selection of appropriate protecting groups, can be readily determined by one skilled in the art. The chemistry of protecting groups can be found, for example, in T. W. Greene and P. G.M. Wuts, "Protective Groups in Organic Synthesis", 3rd Ed., Wiley & Sons, Inc, New York (1999) (ref. 29), which is incorporated herein by reference in its entirety. Reactions can be monitored according to any suitable method known in the art. For example, product formation can be monitored by chromatographic methods such as thin layer chromatography (TLC) or high-performance liquid chromatography (HPLC), or by spectroscopic means, such as nuclear magnetic resonance spectroscopy (e.g., ¹H, ¹³C or¹⁹F), infrared spectroscopy, spectrophotometry (e.g., UV-visible) or mass spectroscopy.

In an exemplary reaction pathway, compounds of the invention can be prepared following the methods outlined in the general synthetic **scheme A:**

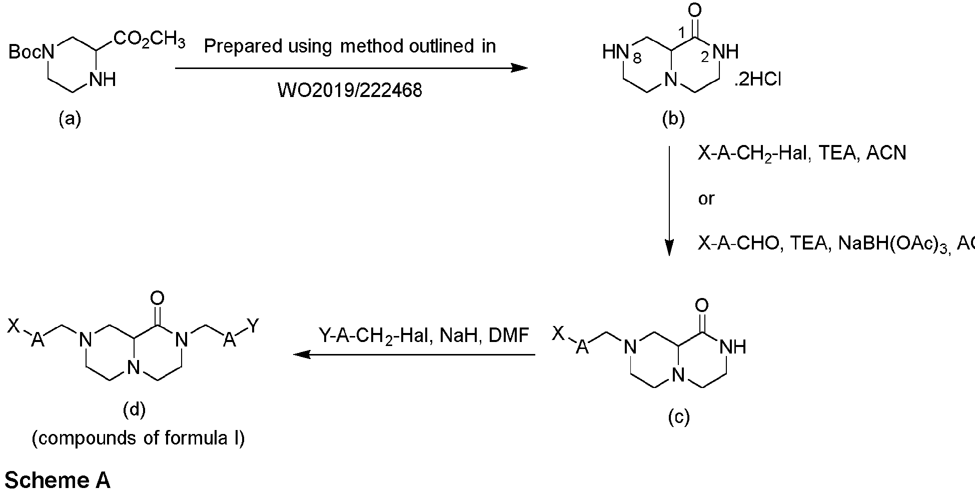

Boc protected piperazine-2-carboxylate (a), which are either commercially available or readily prepared using published chemistry, are transformed into the bicyclic amine (b) via a three-step process outlined in the patent application WO2019/222468. Preferential alkylation of the N8 nitrogen of (b) by either a typical nucleophilic substitution reaction or a classical reductive animation reaction generates intermediate (c). N-alkylation of the amide N2 nitrogen under basic conditions provides compounds (d) of formula (I).

### EXAMPLES

The following examples are offered to illustrate, but not to limit, the claimed invention.

According to the general synthetic schemes A, the following compounds of formula (I), included in the following **table 1** (ID# 1 to 14), below, were prepared:

| **ID** | **Structure** | **ID** | **Structure** |
|---|---|---|---|
| 1 | 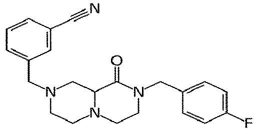 | 10 | 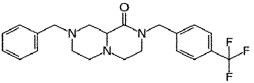 |
| 2 | 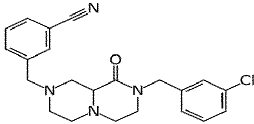 | 11 | 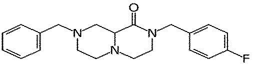 |
| 3 | 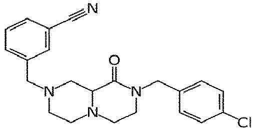 | 12 | 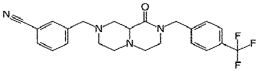 |
| 4 | 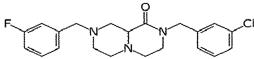 | 13 | 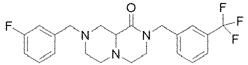 |
| 5 | 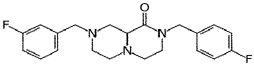 | 14 | 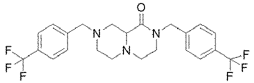 |
| 6 | 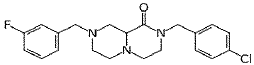 | | |
| 7 | 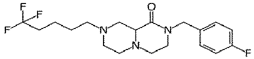 | | |
| 8 | 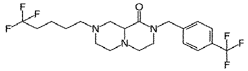 | | |
| 9 | 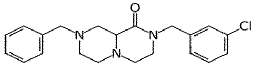 | | |

In the examples below, unless otherwise stated, temperatures are given in degrees Celsius (°C); operations were carried out at room, or ambient temperature (typically a range from 15-25°C); evaporation of solvent was carried out using a rotary evaporator under reduced pressure (typically 4-30mmHg/ with a bath temperature of up to 60°C; and the following conventional abbreviations are also used: mp (melting point), L (liter(s)), mL (millitres), mmol (millimoles), g (grams), mg (milligrams), min (minutes), and h (hours).

### EXAMPLE 1: Synthesis of N8-substitutedhexahydro-2H-pyrazino[1,2-a]pyrazin-1(6H)-ones (b) according to general scheme A.

### 1.1. General procedure for the alkylation of compounds (a) to prepare compounds (b).

Triethylamine (3 eq) was added to a suspension of exahydro-2H-pyrazino[1,2-a]pyrazin-1(6H)-one dihydrochloride (b1, 1 eq) in acetonitrile (10 ml /mmol of b1) at rt. A benzyl bromide derivative (1.1 eq) was then added dropwise and the resulting mixture stirred at rt for 3h. The solvent was removed by rotary evaporation and ethyl acetate (10 ml) added. The organics were washed sequentially with water and brine, then dried (MgSO₄) and filtered. The solvent was removed under reduced pressure to afford an oil which was used without further purification.

### 1.1.1 Preparation of 8-(3-fluorobenzyl)hexahydro-2H-pyrazinof1,2-alpyrazin-1(6H)-one (b1)

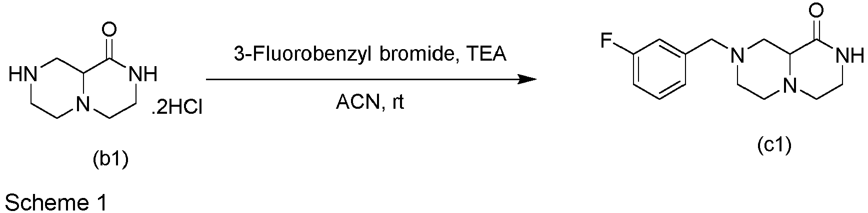

Compound (c1) was prepared using the general procedure described in part 1.1 using 3-fluorobenzyl bromide as the benzyl bromide derivative. Product was a colourless oil. Yield = 82 %

The following intermediates (c2-c5) were prepared using the same synthetic method described in example 1.1. Yields ranged from 67-85%

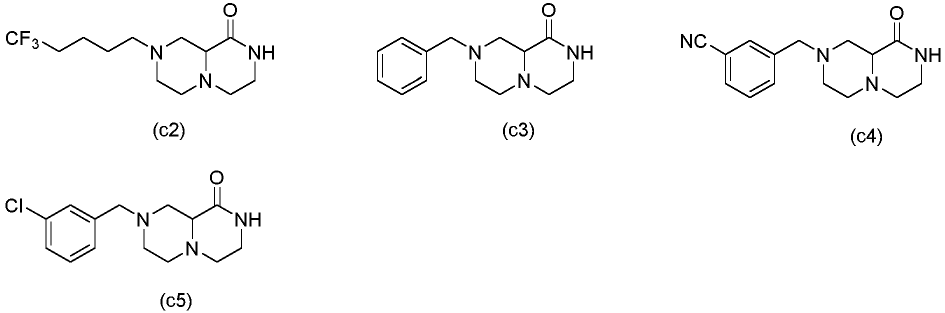

### EXAMPLE 2: Synthesis of compounds of formula (I) (n = 1) according to general scheme A.

### 2.1. General method for the preparation of N2, N8 bis-substituted hexahydro-2H-pyrazino[1,2-alpyrazin-1(6H)-one (d)

NaH (60% in mineral oil, 1.5 eq) was added to a stirring solution of intermediate (c) (1 eq) in anhydrous DMF (0.2M) under Argon at room temperature. After 15 minutes, the desired alkylating reagent was added (1 eq). The reaction mixture was allowed to stir for 3-6 hours at room temperature. Water was added and the solvents removed by lyophilization. DCM was added to the residue and the organics were filtered to remove the inorganic salts.

Concentration of the organics followed by purification by column chromatography (DCM/MeOH; 98.:2) and removal of the solvents afforded the desired product, generally as a colourless viscous oil, or a white solid.

### 2.1.1 Preparation of 8-(3-fluorobenzyl)-2-(3-(trifluoromethyl)benzyl)hexahydro-2H-pyrazinof1,2-alpyrazin-1(6H)-one (13)

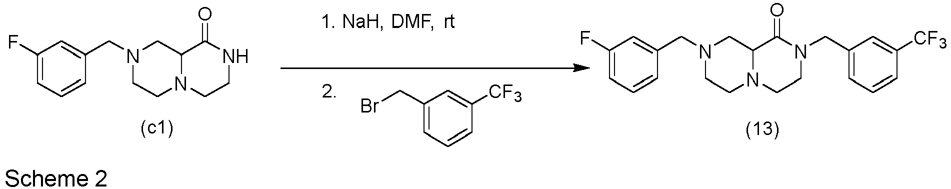

Compound (13) was prepared using the general procedure described in part 2.1, using 3-(trifluoromethyl)benzyl bromide as the alkylating agent. Product was an off white solid. Yield = 37 %

Using the same synthetic method described in example 2.1, the following compounds of formula (I) were prepared:

| Reactant (c) | Alkylating reagent | Compound ID # |
|---|---|---|
| c1 | 4-Fluorobenzyl bromide | 5 |
| c1 | 4-Chlorobenzyl bromide | 6 |
| c1 | 3-Chlorobenzyl bromide | 4 |
| c2 | 4-fluorobenzyl bromide | 7 |
| c2 | 4-(Trifluoromethyl)benzyl bromide | 8 |
| c3 | 3-Chlorobenzyl bromide | 9 |
| c3 | 4-fluorobenzyl bromide | 11 |
| c3 | 4-(Trifluoromethyl)benzyl bromide | 10 |
| c4 | 4-(Trifluoromethyl)benzyl bromide | 12 |
| c4 | 4-fluorobenzyl bromide | 1 |
| c4 | 3-Chlorobenzyl bromide | 2 |
| c4 | 4-Chlorobenzyl bromide | 3 |

### Examples of spectral data for compounds of formula (I):

Compound (8. 2HCl): ¹H NMR (d₆-DMSO, 500 MHz) : δ 7.78 (d, 2H, *J* = 7.8 Hz), 7.53 (d, 2H, *J* = 8.0 Hz), 4.70 (d, 1H, *J* = 14.7 Hz), 4.65 (d, 1H, *J* = 15.3 Hz), 3.88 (d, 1H, *J* = 11.4 Hz), 3.55 (d, 1H, *J* = 11.7 Hz), 3.49-3.41 (m, 2H), 3.40-3.34 (m, 1H), 3.27 (d, 2H, *J =* 9.8 Hz), 3.20-3.08 (m, 2H), 3.07-2.95 (m, 2H), 2.68 (d, 2H, *J=* 12.6 Hz), 2.43-2.32 (m, 2H), 1.91-1.81 (m, 2H), 1.63-1.55 (m, 2H).

Compound (5): ¹H NMR (CDCl₃, 500 MHz) : δ 7.28-7.19 (m, 3H), 7.10-7.05 (m, 2H), 7.03-6.97 (m, 2H), 6.96-6.91 (m, 1H), 4.66 (d, 1H, *J=* 14.6 Hz), 4.37 (d, 1H, *J=* 14.6 Hz), 3.62 (d, 1H, *J=* 13.3 Hz), 3.52 (d, 1H, *J =* 13.4 Hz), 3.50-3.42 (m, 2H), 3.06 (dd, 1H, *J =* 11.5, 4.0 Hz), 2.91-2.85 (m, 2H), 2.82-2.73 (m, 2H), 2.52 (dt, 1H, J = 11.9, 4.3 Hz), 2.38 (dt, 1H, *J* = 11.2, 2.7 Hz), 2.22 (dt, 1H, *J* = 11.0, 2.5 Hz), 2.14-2.09 (m, 1H).

Compound (6): ¹H NMR (CDCl₃, 500 MHz) : δ 7.23-7.16 (m, 3H), 7.10 (d, 2H, J = 8.3 Hz), 7.02-6.97 (m, 2H), 6.88-6.83 (m, 1H), 4.59 (d, 1H, *J=* 14.7 Hz), 4.29 (d, 1H, *J=* 14.7 Hz), 3.55 (d, 1H, *J=* 13.4 Hz), 3.44 (d, 1H, *J =* 13.4 Hz), 3.42-3.35 (m, 2H), 2.98 (dd, 1H, *J* = 11.5, 3.9 Hz), 2.83-2.78 (m, 2H), 2.74-2.66 (m, 2H), 2.45 (dt, 1H, *J=* 12.0, 4.3 Hz), 2.31 (dt, 1H, *J* = 11.2, 2.7 Hz), 2.15 (dt, 1H, *J* =11.0, 2.5 Hz), 2.07-2.00 (m, 1H).

Compound (3): ¹H NMR (CDCl₃, 500 MHz) : δ 7.66 (s, 1H), 7.57-7.52 (m, 2H), 7.43-7.39 (m, 1H), 7.29 (d, 2H, *J=* 8.3 Hz), 7.18 (d, 2H, *J* = 8.4 Hz), 4.65 (d, 1H, *J =* 14.7 Hz), 4.36 (d, 1H, *J =* 14.7 Hz), 3.63 (d, 1H, *J=* 13.7 Hz), 3.56 (d, 1H, *J=* 13.7 Hz), 3.51-3.40 (m, 2H), 3.06 (dd, 1H, *J=* 11.4, 4.0 Hz), 2.92-2.84 (m, 2H), 2.82 (d, 1H, *J=* 10.8 Hz), 2.73 (d, 1H, *J=* 10.9 Hz), 2.54 (dt, 1H, *J=* 11.9, 4.3 Hz), 2.39 (dt, 1H, *J*= 11.2, 2.7 Hz), 2.26 (dt, 1H, *J*= 11.1, 2.6 Hz), 2.14-2.09 (m, 1H).

Compound (2): ¹H NMR (CDCl₃, 500 MHz) : δ 7.66 (s, 1H), 7.58-7.53 (m, 2H), 7.44-7.39 (m, 1H), 7.27-7.21 (m, 3H), 7.15-7.11 (m, 1H), 4.68 (d, 1H, *J =* 14.8 Hz), 4.37 (d, 1H, *J =* 14.8 Hz), 3.64 (d, 1H, *J* = 13.7 Hz), 3.56 (d, 1H, *J =* 13.7 Hz), 3.52-3.41 (m, 2H), 3.08 (dd, 1H, *J =* 11.5, 4.0 Hz), 2.92-2.84 (m, 2H), 2.82 (d, 1H, *J=* 10.8 Hz), 2.73 (dd, 1H, *J=* 10.8, 1.5 Hz), 2.56 (dt, 1H, *J=* 12.0, 4.3 Hz), 2.40 (dt, 1H, *J=* 11.1,2.7 Hz), 2.26 (dt, 1H, *J* = 11.1,2.6 Hz), 2.16-2.10 (m, 1H).

Compound (14): ¹H NMR (CDCl₃, 500 MHz) : δ 7.66-7.55 (m, 4H), 7.45 (d, 2H, *J=* 7.9 Hz), 7.35 (d, 2H, *J=* 7.9 Hz), 4.73 (d, 1H, *J=* 14.9 Hz), 4.48 (d, 1H, *J=* 14.9 Hz), 3.68 (d, 1H, *J=* 13.5 Hz), 3.59 (d, 1H, *J* = 13.5 Hz), 3.52-3.44 (m, 2H), 3.08 (dd, 1H, *J* = 11.5, 3.8 Hz), 2.93-2.88 (m, 2H), 2.83-2.73 (m, 2H), 2.56 (dt, 1H, *J=* 12.0, 4.2 Hz), 2.40 (dt, 1H, *J* = 11.1,2.4 Hz), 2.25 (dt, 1H, *J=* 11.1,2.3 Hz), 2.17-2.11 (m, 1H).

Compound (10): ¹H NMR (CDCl₃, 500 MHz) : δ 7.58 (d, 2H, *J=* 8.0 Hz), 7.38-7.23 (m, 7H), 4.73 (d, 1H, J = 15.1 Hz), 4.47 (d, 1H, *J=* 15.1 Hz), 3.67 (d, 1H, *J=* 12.6 Hz), 3.54 (d, 1H, *J* = 13.2 Hz), 3.52-3.45 (m, 2H), 3.07 (dd, 1H, *J =* 11.5, 3.5 Hz), 2.94-2.86 (m, 2H), 2.83-2.76 (m, 2H), 2.55 (dt, 1H, *J =* 11.9, 4.0 Hz), 2.45-2.36 (m, 1H), 2.26-2.19 (m, 1H), 2.16-2.10 (m, 1H).

Compound (9): ¹H NMR (CDCl₃, 500 MHz) : δ 7.33-7.29 (m, 4H), 7.27-7.22 (m, 4H), 7.14-7.11 (m, 1H), 4.69 (d, 1H, *J=* 14.7 Hz), 4.35 (d, 1H, *J=* 14.7 Hz), 3.66 (d, 1H, *J=* 13.1 Hz), 3.53 (d, 1H, *J=* 12.7 Hz), 3.51-3.43 (m, 2H), 3.06 (dd, 1 H, *J* = 11.5, 3.6 Hz), 2.93-2.86 (m, 2H), 2.82-2.76 (m, 2H), 2.54 (dt, 1H, *J=* 11.8, 4.0 Hz), 2.43-2.36 (m, 1H), 2.25-2.18 (m, 1H), 2.16-2.10 (m, 1H).

Compound (12. 2HCl): ¹H NMR (d₆-DMSO, 500 MHz) : δ 8.18-8.04 (m, 1H), 8.00-7.90 (m, 2H), 7.73 (d, 2H, *J* = 7.9 Hz), 7.70 (t, 1H, *J* = 7.8 Hz), 7.47 (d, 2H, *J* = 7.9 Hz), 4.61 (s, 2H), 4.53-4.32 (m, 2H), 3.84-3.59 (m, 4H), 3.50-3.27 (m, 3H), 3.27-3.18 (m, 1H), 3.15-2.93 (m, 4H).

Compound (11. 2HCl): ¹H NMR (d₆-DMSO, 500 MHz) : δ 7.67-7.62 (m,2H), 7.50-7.45 (m, 3H), 7.31-7.26 (m, 2H), 7.17 (t, 2H, *J* = 8.8 Hz), 4.53 (d, 1H, *J* = 14.7 Hz), 4.46 (d, 1H, *J* = 14.8 Hz), 4.38 (s, 2H), 3.75-3.64 (m, 1H), 3.59-3.42 (m, 1H), 3.41-3.30 (m, 2H), 3.23-315 (m, 1H), 3.14-2.94 (m, 4H), 2.84-2.71 (m, 1H), 2.70-2.58 (m, 1H).

Compound (7. 2HCl): ¹H NMR (d₆-DMSO, 500 MHz) : δ 7.33-7.27 (m, 2H), 7.18 (t, 2H, *J* = 8.8 Hz), 4.57 (d, 1H, *J* = 14.6 Hz), 4.47 (d, 1H, *J* = 14.7 Hz), 3.83 (d, 1H, J = 11.8 Hz), 3.56-3.45 (m, 2H), 3.44-3.30 (m, 1H), 3.26-3.16 (m, 3H), 3.15-3.05 (m, 2H), 3.04-2.91 (m, 2H), 2.80-2.56 (m, 2H), 2.40-2.27 (m, 2H), 1.87-1.76 (m, 2H), 1.53 (p, 2H, *J* = 7.5 Hz).

Compound (4. 2HCl): ¹H NMR (d₆-DMSO, 500 MHz) : δ 7.66-7.57 (m, 1H), 7.56-7.50 (m, 1H), 7.49-7.44 (m, 1H), 7.42-7.33 (m, 3H), 7.32-7.29 (m, 1H), 7.25-7.20 (m, 1H), 4.58-4.47 (m, 2H), 4.45-4.34 (m, 2H), 3.75-3.65 (m, 1H), 3.48-3.36 (m, 1H), 3.35-3.28 (m, 1H), 3.27-2.93 (m, 6H), 2.90-2.63 (m, 2H).

Compound (1. 2HCl): ¹H NMR (d₆-DMSO, 500 MHz) : δ 8.15 (brs, 1H), 7.99 (d, 1H, *J* = 7.3 Hz), 7.95 (d, 1H, *J* = 7.8 Hz), 7.69 (t, 1H, *J* = 7.8 Hz), 7.30 (dd, 2H, *J* = 8.2, 6.0 Hz), 7.17 (t, 2H, *J* = 8.9 Hz), 4.53 (d, 1H, *J* = 15.0 Hz), 4.49 (d, 1H, *J* = 15.0 Hz), 4.46-4.34 (m, 2H), 3.74-3.49 (m, 2H), 3.46-3.36 (m, 1H), 3.34-3.26 (m, 1H), 3.26-2.97 (m, 5H), 2.87-2.65 (m, 2H).

Compound (13): ¹H NMR (CDCl₃, 500 MHz) : δ 7.55-7.50 (m, 1H), 7.48 (s, 1H), 7.45 (d, 2H, J = 4.4 Hz), 7.28-7.23 (m, 1H), 7.10-7.05 (m, 2H), 6.96-6.91 (m, 1H), 4.75 (d, 1H, *J* = 14.8 Hz), 4.46 (d, 1H, *J* = 14.8 Hz), 3.63 (d, 1H, *J* = 13.4 Hz), 3.52 (d, 1H *J* = 13.4 Hz), 3.50-3.45 (m, 2H), 3.08 (dd, 1H, *J* = 11.4, 3.5 Hz), 2.93-2.88 (m, 2H), 2.81 (d, 1H, J = 10.8 Hz), 2.76 (d, 1H, *J* = 11.0 Hz), 2.56 (dt, 1H, *J* = 11.9, 4.1 Hz), 2.40 (dt, 1H, *J* = 11.1, 2.3 Hz), 2.23 (dt, 1H, *J* = 10.9, 2.0 Hz), 2.15-2.10 (m, 1H).

### Example 2.1.2: General method for the preparation of N2, N8 bis-substituted hexahydro-2H-pyrazinof1,2-alpyrazin-1(6H)-one (d), wherein the two groups at N2 and N8 are identical.

To a stirring solution of (b1) in DMF (1 mL/0.1 mmol of b1) was added NaH (4.5 eq). After 15 minutes, a solution 4-(trifluoromethyl) benzyl bromide (2.2 eq) in DMF (0.5 mL) was added dropwise and the solution stirred for another hour. The solution was cooled to 0 °C, and water (2 ml/ 0.1 mmol) was added, and the solution was frozen. Solvent was removed by lyophilization overnight. The organics were dissolved in DCM and purified by column chromatography (DCM/MeOH 92:8). Removal of solvent afforded the desired product.

### 2.1.3 Preparation of 8-(4-trifluoromethylbenzyl)-2-(4-(trifluoromethyl)benzyl)hexahydro-2H-pyrazino[1,2-a]pyrazin-1(6H)-one (14).

Compound (14) was prepared using the general procedure described in part 2.1.2, using 4-(trifluoromethyl)benzyl bromide as the alkylating agent. Product was an off white solid. Yield = 52 %

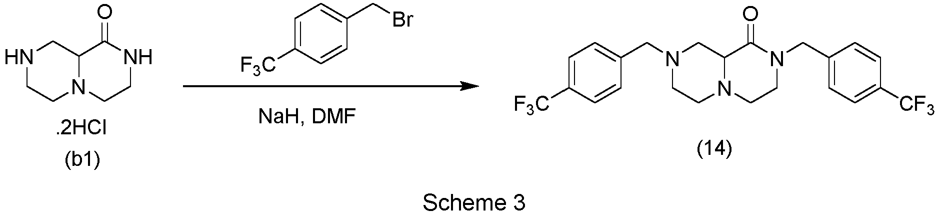

### EXAMPLE 3: Synthesis of HCl salts of compounds of formula (I).

3.1. General procedure for the preparation of hydrochloride salts of compounds of formula (I). To compound of formula (I) was added a solution of HCl in methanol (1.25M, 5 eq) at RT. The solution was stirred for 1h, then the organics were removed under reduced pressure. If needed, diethyl ether was added to aid precipitation of the salt which was collected by filtration and dried in vacuo at 45 °C.

### 3.1.1. Preparation of 8-(3-fluorobenzyl)-2-(3-(trifluoromethyl)benzyl)hexahydro-2H-pyrazinof1,2-alpyrazin-1(6H)-one (13. 2HCl)

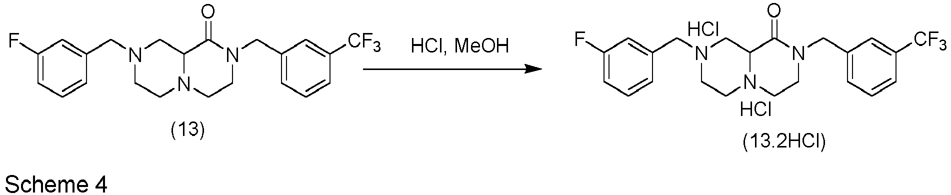

The titled compound was obtained using the general procedure described in example 3.1 Product (formula I. 2HCl) was obtained in quantitative yield as a white solid.

### EXAMPLE 4: Biological experiments

Experiments were performed as shown below for the compounds of formula (I) of present invention prepared in the examples described above herein.

### 4.1. Method used to identify selective human CIpP activators using genetically modified yeast.

### 4.1.1. Preparation of yeast, with or without the presence of human CIpP, that have several efflux pumps down regulated.

### Strain construction

Yeast cells expressing hCIpP were constructed by using the *Modular Cloning* (MoClo) method (Lee ME, DeLoache WC, Cervantes B, Dueber JE. A Highly Characterized Yeast Toolkit for Modular, Multipart Assembly. ACS Synth Biol. 2015 Sep 18;4(9):975-86. doi: 10.1021/sb500366v. Epub 2015 May 1. PMID: 25871405). This technique allows to directionally assemble several DNA fragments into one single DNA piece. It is based on plasmid libraries that share the same DNA backbone but differ in the type of the insert. The assembly of the final cassette is based on the *"Golden Gate Cloning"* method. Human *CLPP* gene sequence (codon-optimized for yeast translation) was amplified with well-designed primers from a plasmid kindly provided by Dr. Michael David Tyers (University of Montreal). Once amplified, a MoClo reaction was performed to obtain the final integrative cassette. This cassette was composed as follows:
- Type 1: *ConLS* 3' connector.
- Type 2: *TDH3* gene promoter (strong and constitutive expression).
- Type 3: Amplified human *CLPP* gene.
- Type 4: *ADH1* gene terminator.
- Type 5: ConR1 5' connector.
- Type 6: *LEU2* gene as selectable marker for yeast.
- Type 7: 3' HO gene homology sequence for yeast integration.
- Type 8a: *KanR-ColE1* sequence for bacterial amplification and antibiotic resistance.
- Type 8b: 5' HO gene homology sequence for yeast integration.

After plasmid assembly reaction and subsequent transformation in *DH5α Escherichia coli,* large amount of the plasmid was obtained by *Maxiprep.* Digestion patterns with several restriction enzymes were carried out to confirm the success of the MoClo system. An extra restriction enzyme reaction (*Notl*) was performed to linearize the DNA fragment and deplete the bacterial part. Linearization gave rise to a lineal fragment flanked by 5' and 3' homology sequences to the HO gene, which further served for chromosomic integration at the mutated HO locus.

The used yeast strain contains the following background:
*MATa trp1-1 leu2-3,112 his3-11,15 ura3-1 ade2-1 can1-100 yrs1::HIS3 yrr1::TRP1 pdr1::hisG pdr3::hisG.*

These cells contain mutants in *YRS1, YRR1, PDR1* and *PDR3* genes, which are involved in polydrug and pleiotropic resistance networks. These mutants significantly reduce the capability of the yeast to remove xenobiotic compounds.

Yeast cells were transformed with the linearized cassette and transformant colonies were selected on minimum media without leucine. Independent clones were checked by PCR and positive ones were stored at -80 °C until use.

### 4.1.2. Screening of compounds to identify activators of human CIpP.

The yeast (both HsCpIP+ and HsCIpP-) are incubated in petri dishes with YPD (Yeast extract peptone dextrose) at 25 °C overnight, after which a colony is isolated, suspended in a flask with fresh YPD and incubating again under the same conditions but with agitation. The culture was then diluted with YPD until an absorbance reading of 0.1 was achieved at 600 nM. 100 µl of this solution was then placed into the appropriated wells of a standard 96-well plate. Stock solutions (DMSO, 10 mM) of the compounds to be tested are diluted in YPD to 2X of their final concentrations to be tested, and 100 µl added to the appropriate wells. 100 µl of YPD was added to wells designated as the negative control wells, while ONC206 (0.5 µM) was used as the positive control. Once all compounds were added to the plate, an initial T = 0 absorbance value was recorded at 600 Nm. The plate was then incubated at 25 °C with gentle shaking for 24 h. The plates can either be read at multiple time points using a TECAN Spark^{®} Multimode Microplate Reader, or at the end of the experiment using a standard spectrophotometer. The 50% growth inhibition concentration (GI50) was calculated using the GraphPad 5.0 program.

Table 2 below sets forth potencies of representative compounds of the invention to inhibit yeast growth via modulating HsCIpP. None of the compounds represented in table 2 inhibited the growth of yeast not possessing HsClpP at concentrations up to 10000 nM.

**Table 2: Growth inhibition (GI50) values for compounds of the present invention in yeast containing HsCIpP, and yeast without HsCIpP.**

| ID# (table 1) | Yeast + HsCIpP GI50 (nM) | Yeast - HsCIpP GI50 (nM) |
|---|---|---|
| ONC201 | 10000 | >10000 |
| ONC206 | 4560 | >10000 |
| 1 | 150 | >10000 |
| 2 | 120 | >10000 |
| 3 | 65 | >10000 |
| 4 | 210 | >10000 |
| 5 | 140 | >10000 |
| 6 | 60 | >10000 |
| 7 | 2050 | >10000 |
| 8 | 1650 | >10000 |
| 9 | 690 | >10000 |
| 10 | 180 | >10000 |
| 11 | 620 | >10000 |
| 12 | 75 | >10000 |
| 13 | 40 | >10000 |
| 14 | 2580 | >10000 |

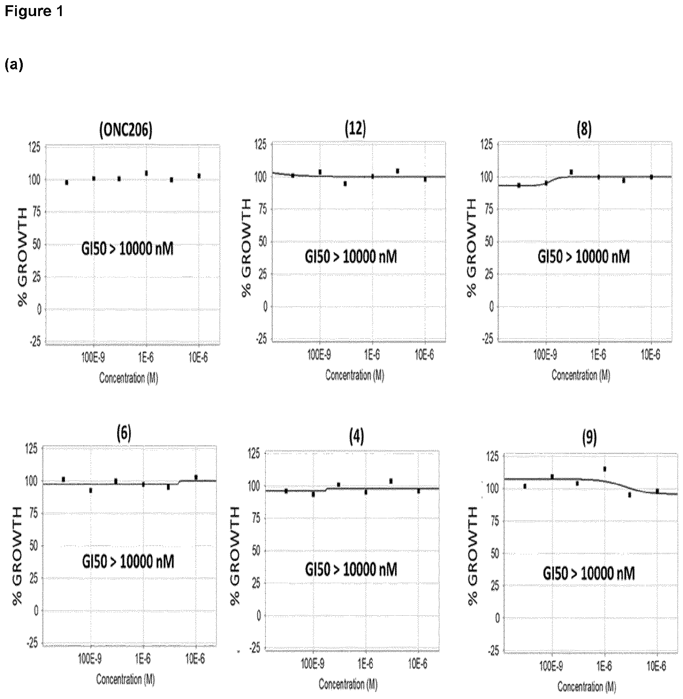

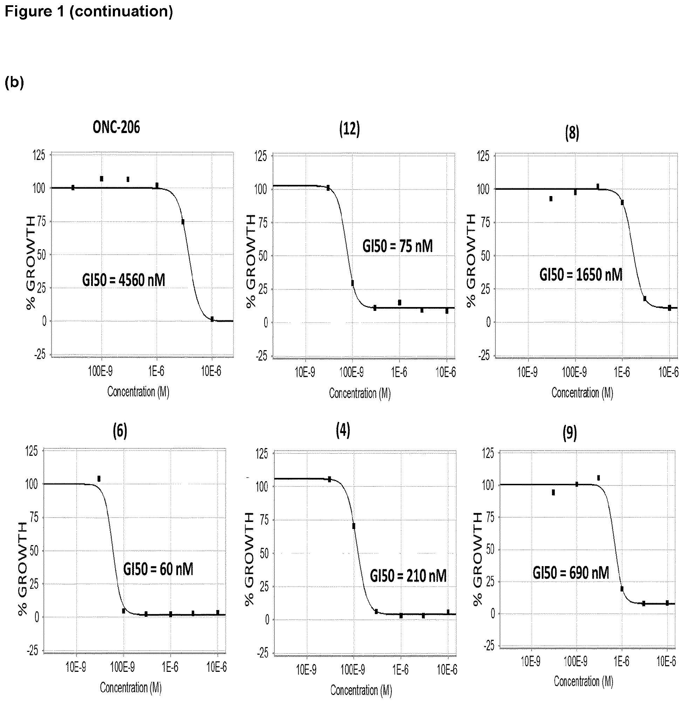

Moreover, Fig. 1 shows, how compounds of the invention selectively inhibit the growth of yeast that contain HsClpP and are not toxic to yeast without HsClpP present. Furthermore, several compounds of the invention are more potent in this assay compared to both of the ClpP agonists in human clinical trials, ONC-201 and ONC-206

### 4.1.3. The effects of the compounds of the present invention on the cell viability of various human and murine derived cancer cell lines.

### Cell lines.

DU-145 [American Type Culture Collection (ATCC) reference number HTB-81]. This a human prostate cancer cell line derived from a metastatic site (brain) of a 68-year-old male.

PANC-1 [American Type Culture Collection (ATCC) reference number CRL-1469]. This a human cancer cell line derived from the pancreas of a 56-year-old male.

MDA-MB-231 [American Type Culture Collection (ATCC) reference number HTB-26]. This a human breast cancer cell line derived from a metastatic site from a 51-year-old female.

4T1 [American Type Culture Collection (ATCC) reference number CRL-2539]. This a mouse epithelial breast cancer cell line classified as being triple negative and mimics human breast cancer; stage IV.

U2 OS [American Type Culture Collection (ATCC) reference number HTB-96]. This is a human bone cancer (a moderately differentiated sarcoma) cell derived from a female osteosarcoma patient.

### MTT Cell viability assay.

Cell viability was assayed by measuring the mitochondrial reduction of the tetrazolium salt 3-(4,5-methyltiazol-2yl)-2,5diphenyl-tetrazolium bromide] (MTT) (Applichen; A2231,0010) [Mosmann T. J Immunol Methods. 1983 16;65(1-2):55-63 (ref. 36); Chuan Y-C et al 2010. Oncogene. 29 (10), 1531-1542 (ref. 37)]. Cells were seeded in 96-well plates in medium (either RPMI, DMEM or McCoys depending on the recommendations from the ATCC) supplemented with 10% FBS (Lonza Cat. N°: 14-502F), 2mM L-Glut (Lonza Cat. N°: BE17-605E) and 100U/ml PEST (Lonza Cat. N°: DE17-605E) at a cell density of 1000-5000 cells/well (96 well plate). Cells were seeded at a density according to their exponential growth phase (normally 1000 to 5000/well). The cells were cultured at 37 °C in the dark in air containing 5% CO₂. After 24h either vehicle (0.05% DMSO), or drug, was added to culture medium at the indicated concentrations (10 to 0,01microM). After another 72 h, MTT (0.3 mg/ml) was added to cells, followed by incubation for 2-4h at 37 °C in the dark. The medium was then removed, and the precipitated formazan was solubilized by the addition of DMSO (200 µL).

The optical density was measured at 595 nm with the iMark Microplate Reader (BioRad, USA). Data was expressed as percent growth above the level in controls and the results in figures were plotted as means ± SEM of each test point from 3 replicates.

Statistical analysis. The concentration of drug that provokes 50% reduction of cell viability (IC₅₀) was determined by using non-linear regression analysis and data were fitted to a log concentration vs normalized response curve in GraphPad software v5 (San Diego, CA).

Table 3 below sets forth potencies of representative compounds of the invention in a variety of human or mouse derived cancer cell lines (values are averages of at least two independent experiments).

**Table 3: Compounds of the invention (formula I) reduce the cell viability in a collection of human or mouse cancer cell lines.**

| ID# (Table 1) | IC₅₀ (nM) 4T1 48 H | IC₅₀ (nM) MDA-MB-231 72 H | IC₅₀ (nM) U2 OS 72 H | IC₅₀ (nM) DU-145 72 H | IC₅₀ (nM) PANC1 72 H |
|---|---|---|---|---|---|
| ONC201 | 2215 | 2285 | 4800 | 1710 | 1720 |
| ONC206 | 600 | 600 | 800 | 370 | 300 |
| 1 | 103 | 120 | 220 | 200 | 195 |
| 2 | 165 | 145 | 250 | 250 | 140 |
| 3 | <100 | <100 | <100 | <100 | <100 |
| 4 | 330 | 450 | 500 | 850 | 335 |
| 5 | 190 | 240 | 400 | 300 | 250 |
| 6 | 150 | 190 | 300 | 250 | 190 |
| 7 | 340 | 430 | 500 | 500 | 330 |
| 8 | 310 | 400 | 2000 | - | 350 |
| 9 | 930 | 935 | 1140 | 1080 | 830 |
| 10 | 650 | 635 | 1000 | 640 | 640 |
| 11 | 515 | 490 | 800 | 410 | 600 |
| 12 | <100 | <100 | 200 | 100 | <100 |
| 13 | 170 | 190 | 300 | 250 | 165 |
| 14 | 2220 | - | - | - | 240 |

| | | | | | |
|---|---|---|---|---|---|
| - represents compound not tested. | | | | | |

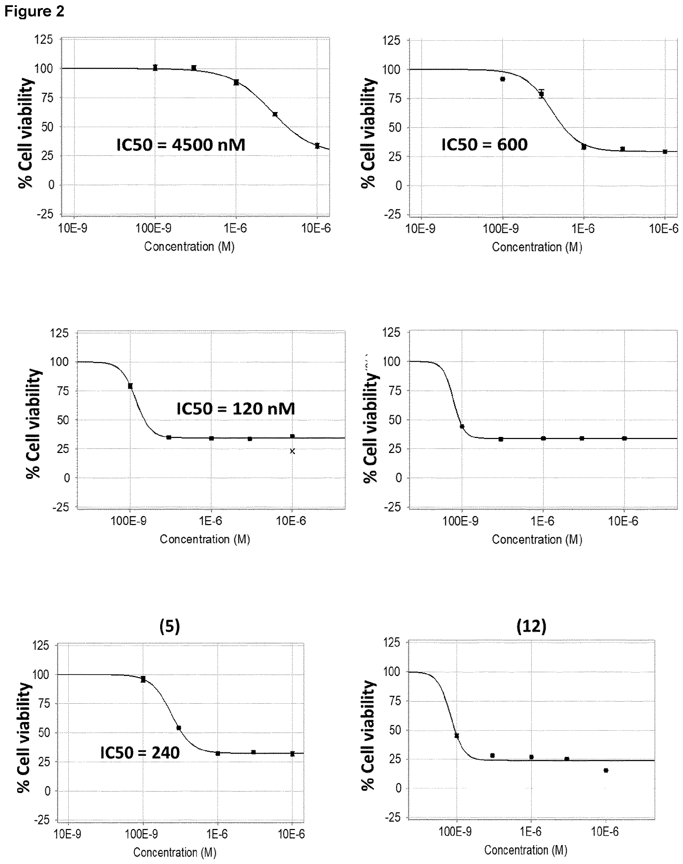

Moreover, Figure 2 shows that several compounds of the invention are more potent against MDA-MB-231 cells, than the two CIpP agonists currently in human clinical trials, ONC-201 and ONC-206. According to the clinical trial website www.clinicaltrial.gov accessed on March 9th 2023, ONC-201 is in 9 active or recruiting clinical trials related to cancer, and ONC-206 is in 2 active or recruiting clinical trials related to cancer.

### 4.1.4. The effects of the compounds of the present invention on the cell growth of various human and murine derived cancer cell lines.

### SRB cell growth assay.

Sulforhodamine B (SRB) binds selectively to cellular proteins and can therefore be used to measure the effects of compounds on cellular growth. The method described here (modified from Houghton, P., et. al., Methods 42 (2007) 377-387) uses sulforhodamine B (SRB) as a means to analyze the effects of compounds on adherent cells using a 96-well format.

In order to measure the cell population at the time zero (Tz) a plate of cells was stained with SRB 24 hours after seeding. The cell monolayers are fixed with cold 5% (w/v) trichloroacetic acid for 60 minutes at 4°C. The excess of trichloroacetic acid is removed by washing five times with distilled water. Sulforhodamine B (SRB) solution [50 µL at 0.4%(w/v) in 1% acetic acid] is added to each well and plates are incubated for 8 minutes at room temperature. The excess dye is removed by washing five times with 1% (v/v) acetic acid. The bound stain is subsequently solubilized with (100 µL) 10 mM trizma base and the absorbance is read on automated plate reader at a wavelength of 550 nm.

Compounds at various concentrations were added to an identical cell-seeded (non-treated) plate and incubated at 37°C (5% CO2) for an additional 48 hours. Cells were fixed and stained following the same protocol. The measurement of the cell population after 72 hours in the presence of the drug is (Ti). Several wells in the same plate were treated with the control vehicle. The resulting measurements give the control growth value (C) after 72 h.

Using the absorbance measurements, time zero (Tz), control growth (C), and test growth in the present of the compound (Ti) the percentage growth inhibition is calculated as:
[(Ti-Tz)/(C-Tz)] x 100 for concentrations for which Ti >/= Tz
[(Ti-Tz)/Tz] x 100 for concentrations for which Ti <Tz

### Statistical analysis.

Based on these values for each test concentration, the following parameters can be calculated:
- Growth inhibition concentration (Gl): concentration of compound required to reduce growth by 50%
- Total growth inhibition concentration (TGI): concentration of compound required to reduce growth by 100%
- Lethal concentration (LC): concentration of compound required to kill 50% of the initial cell population.

These values were determined using GraphPad software v5 (San Diego, CA).

**Table 4: Compounds of the invention (formula I) reduce the ability of MDA-MB-231 cells to grow.**

| | MDA-MB-231 72 H | | | |
|---|---|---|---|---|
| ID# (Table 1) | GI₅₀ (nM) | TGI₅₀ (nM) | LC₅₀ (nM) | Result |
| ONC201 | 2500 | >10000 | >10000 | Cytostatic |
| ONC206 | 730 | >10000 | >10000 | Cytostatic |
| 3 | <100 | >10000 | >10000 | Cytostatic |
| 12 | <100 | >10000 | >10000 | Cytostatic |
| 13 | 420 | >10000 | >10000 | Cytostatic |

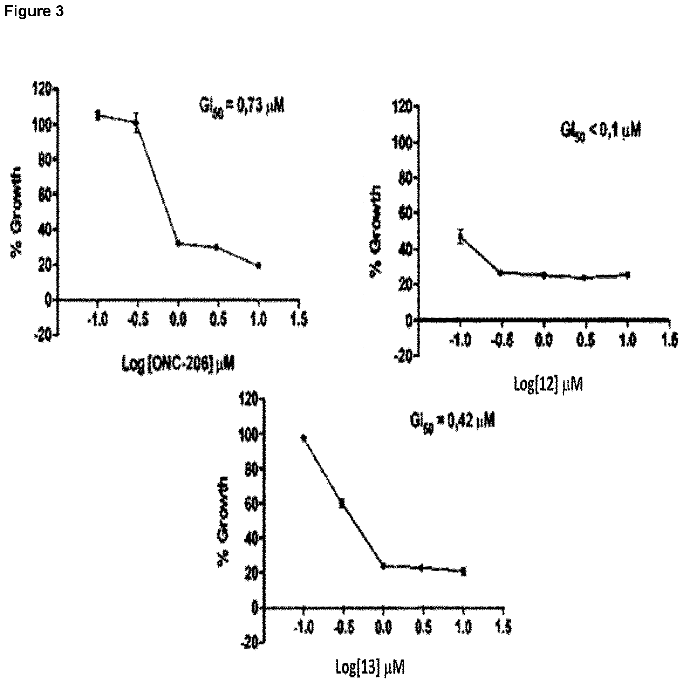

Additionally, Fig. 3 also shows that compounds (12) and (13) of the present invention reduce the growth of MBA-MB-231 cells, and are cytostatic agents.

### 4.1.5. Effect of compounds of the present invention on tumours induced in immunocompetent mice using the murine TNBC cell line 4T1.

4T1 syngeneic tumours were induced in female immunocompetent Balb/c mice by inject 12500 4T1 cells (passage# <7, in PBS, 100 µL) into their left inguinal mammary fat pad. Once tumour volumes [measure using the formula V = 0.5(length x width²)] reached 100 m³, treatment with either vehicle, or compound in vehicle, was began. Compounds were administered orally (PO) at a constant dose (typically 25 mg/kg) three times per week (Monday, Wednesday and Friday). Compounds (as ether their free base or salt) were formulated as either solutions or homogenous suspensions in a vehicle of 10% Cremaphor RH40 + 90% saline for injection. Formulations were prepared at concentrations (mg/mL) = Dose/5 (e.g. a dose of 25 mg/Kg used a formulation of 5 mg/ml). Once treatments began, tumour volumes and body weights were recorded on each treatment day. Treatments were continued until 25-28 days after first treatment day, after which the mice were sacrificed. Animals whose tumour reached >1500 m³ during the experiment were also sacrificed.

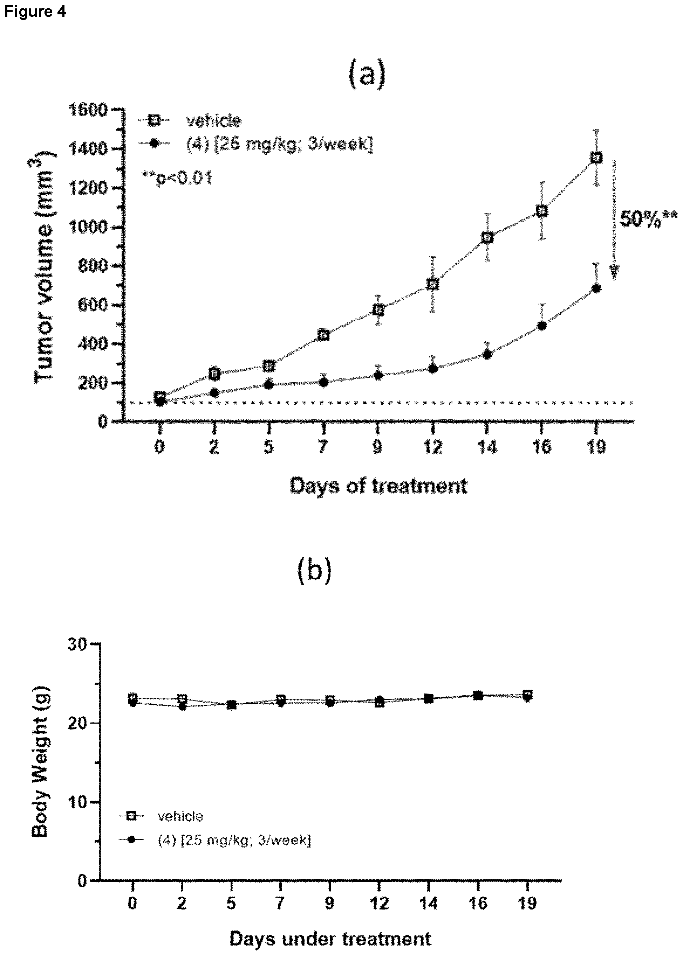

Figure 4a shows the effects of treating mice bearing established 4T1 tumours with either vehicle (10% creamphor in saline), or with 25 mg/kg of compound (4), three time per week.

Tumour growth was significantly reduced (50%, p < 0.01) in those mice treated with compound (4), compared to animals treated with just vehicle. Also, treatment with compound (4) caused no significant changes in body weights during the experimental period (Figure 4b).

### References

1. Mabanglo, MF., et al., Substrates and interactors of ClpP protease in the mitochondria, Curr. Opin. Chem. Biol., 2022, feb 66:102078; doi: 10.1016/j.cbpa.2021.07.003 epub 2021 Aug 23.
2. Wang, P., et al., Aberrant human ClpP activation disturbs Mitochondrial proteome homeostasis to suppress pancreatic ductal adenocarcinoma, Cell Chem. Biol., 2022, 29, 1396-1408.
3. Luo, B., et al., Human ClpP protease, a promising therapy for diseases of mitochondrial dysfunction., Drug Dis.Today., 2021 26(4), 968-981.
4. Mabanglo, MF., et al., Substrates and interactors of ClpP protease in the mitochondria, Curr. Opin. Chem. Biol., 2022, feb 66:102078; doi: 10.1016/j.cbpa.2021.07.003 epub 2021 Aug 23.
5. Wong, K. S., et al., Acyldepsipeptide Analogs Dysregulate Human Mitochondrial HsClpP Protease Activity and Cause Apoptotic Cell Death. Cell Chem. Biol. 2018, 25, 1017-1030.e1019.
6. Prabhu, V.V., et al., ONC201 and impridones: Anti-cancer compounds with clinical efficacy, Neoplasia,2020, 22(12), 725-744.
7. Fatima, N., et al., The ClpP activator, TR-57, is highly effective as a single agent and in combination with venetoclax against CLL cells in vitro, BioRxiv preprint doi.org/10.1101/2022.03.07.483345.

## Claims

1. A compound of formula (I), or a pharmaceutically acceptable salt or solvate thereof: 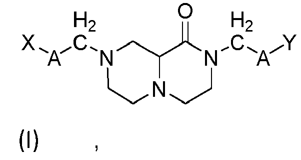 wherein
A is independently selected from the group consisting of: C₁-C₆ alkyl or is absent;
X and Y are independently selected from the group consisting of C₁-C₆ haloalkyl, aromatic carbocyclic 6-membered ring, or an aromatic heterocyclic 5- or 6-membered ring comprising one or more heteroatoms selected from the group consisting of O, N and S, wherein said aromatic carbocyclic ring, or said aromatic heterocyclic ring, is unsubstituted or substituted at one or more atoms of C, with a R⁷ substituent;
R⁷ substituent is independently selected from the group consisting of: H, halogen, C₁-C₆ haloalkyl, -O-C₁-C₆ alkyl, -O-C₁-C₃ haloalkyl, cyano, C₁-C₆ alkyl and -C(O)OR⁸;
R⁸ is independently selected from the group consisting of: C₁-C₆ alkyl, C₃-C₆ cycloalkyl or C₁-C₆ aminoalkyl:

2. A compound of formula (I), or a pharmaceutically acceptable salt or solvate thereof, according to claim 1, wherein:
A is independently selected from the group consisting of: C₁-C₆ alkyl or is absent;
X and Y are independently selected from the group consisting of C₁-C₆ haloalkyl, aromatic carbocyclic 6-membered ring, or an aromatic heterocyclic 5- or 6-membered ring comprising one or more heteroatoms selected from the group consisting of O, N and S, wherein said aromatic carbocyclic ring, or said aromatic heterocyclic ring, is unsubstituted or substituted at one or more atoms of C, with a R⁷ substituent;
wherein R⁷ substituent is independently selected from the group consisting of: H, halogen, C₁-C₆ haloalkyl, -O-C₁-C₆ alkyl, -O-C₁-C₂ haloalkyl, cyano and C₁-C₆ alkyl;

3. A compound of formula (I), or a pharmaceutically acceptable salt or solvate thereof, according to claims 1 or 2, wherein:
A is independently selected from the group consisting of: C₁-C₃ alkyl or is absent;
X and Y are independently selected from the group consisting of C₁-C₃ haloalkyl, aromatic carbocyclic 6-membered ring, or an aromatic heterocyclic 5-membered ring comprising one or more heteroatoms selected from the group consisting of O, N and S, wherein said aromatic carbocyclic ring, or said aromatic heterocyclic ring, is unsubstituted or substituted at one or more atoms of C, with a R⁷ substituent;
wherein R⁷ substituent is independently selected from the group consisting of: H, F, Cl, Br, C₁-C₃ haloalkyl, -O-C₁-C₆ alkyl, -O-CF₃, cyano and C₁-C₆ alkyl;

4. A compound or pharmaceutical acceptable salt or solvate thereof, wherein the compound is selected from:

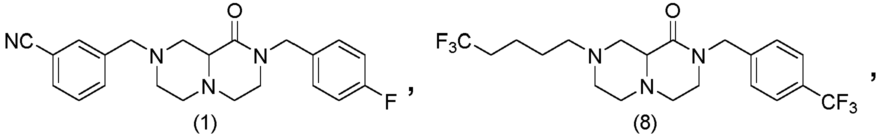

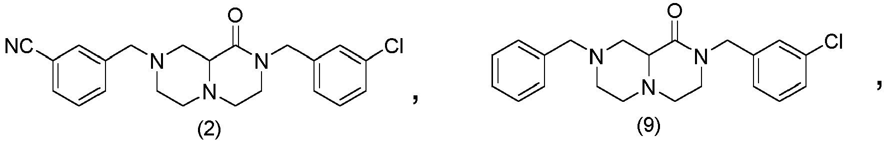

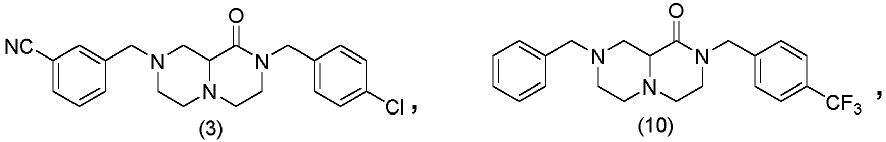

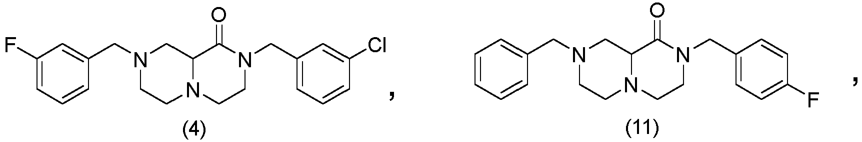

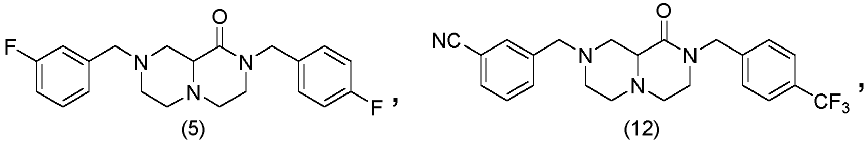

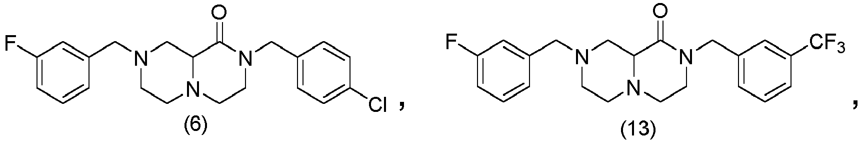

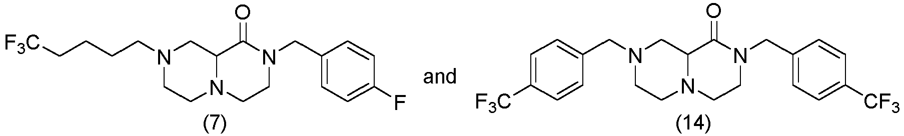

5. A compound of formula (I) or pharmaceutical acceptable salt or solvate thereof, according to any of the claims 1 to 4, wherein the compound, the salt, or the solvate comprise stereoisomers, polymorphs, isotopes, or in the form of complexes with antibodies.

6. A pharmaceutical composition comprising at least one compound of formula (I), according to any of claims 1 to 5, and at least one pharmaceutically acceptable excipient or carrier.

7. A pharmaceutical composition according to claim 6, further comprising at least one additional active compound or agent selected from the group consisting of a chemotherapeutic, a cell therapy, an immunotherapeutic or an antibiotic.

8. A compound of formula (I), according to any of claims 1 to 5, for use as a medicament.

9. A compound of formula (I), according to any of claims 1 to 5, for use in the prevention and/or treatment of cancer.

10. A compound for use according to claim 9, wherein the cancer is selected from the group consisting of: sarcoma, breast cancer, prostate cancer, head and neck cancer, brain tumour, colorectal cancer, lung cancer, pancreatic cancer, cervical cancer, ovarian cancer, gastric cancer, renal cell carcinoma, melanoma, endometrial cancer, hepatocellular carcinoma, chronic myelogenous leukaemia, acute myelogenous leukaemia, cutaneous T-cell lymphoma, Hodgkin's disease, anaplastic large-cell lymphoma and Burkitt's lymphoma.

11. A compound for use according to any of claims 9 or 10, wherein said compound is administered together, prior to, or subsequently to radiotherapy, immunotherapy or chemotherapy.

12. A pharmaceutical composition according to any of claims 6 or 7, for use in the prevention and/or treatment of cancer.

13. A pharmaceutical composition for use according to claim 12, wherein the cancer is selected from the group consisting of: sarcoma, breast cancer, prostate cancer, head and neck cancer, brain tumour, colorectal cancer, lung cancer, pancreatic cancer, cervical cancer, ovarian cancer, gastric cancer, renal cell carcinoma, melanoma, endometrial cancer, hepatocellular carcinoma, chronic myelogenous leukaemia, acute myelogenous leukaemia, cutaneous T-cell lymphoma, Hodgkin's disease, anaplastic large-cell lymphoma and Burkitt's lymphoma.
